(19) Europäisches Patentamt / European Patent Office / Office européen des brevets

(11) **EP 4 582 114 A1**

(12) **EUROPEAN PATENT APPLICATION**

(43) Date of publication:
**09.07.2025 Bulletin 2025/28**

(21) Application number: **25150030.2**

(22) Date of filing: **02.01.2025**

(51) International Patent Classification (IPC):
**A61M 1/02** (2006.01)     **A61M 1/36** (2006.01)

(52) Cooperative Patent Classification (CPC):
**A61M 1/0218; A61M 1/0231;** A61M 1/362264;
A61M 1/3633; A61M 1/3693; A61M 2202/0429

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **04.01.2024 US 202463617641 P**

(71) Applicant: **Fenwal, Inc.
Lake Zurich, IL 60047 (US)**

(72) Inventors:
• **KARLOVSKY, Daniel M.
Lake Zurich, 60047 (US)**
• **ROXAS, James D.
Lake Zurich, 60047 (US)**

(74) Representative: **Maikowski & Ninnemann
Patentanwälte Partnerschaft mbB
Postfach 15 09 20
10671 Berlin (DE)**

(54) **SYSTEMS AND METHODS FOR EXPELLING AIR FROM CONTAINERS**

(57) Systems and methods for processing whole blood are disclosed. The system includes a reusable separation device and a disposable fluid circuit configured to expel air from multiple product containers.

FIG. 1

EP 4 582 114 A1

**Description**

CROSS-REFERENCE TO RELATED APPLICATIONS

This application claims the benefit of and priority to U.S. Provisional Patent Application Serial No. 63/617,641, filed January 4, 2024, the disclosure of which is incorporated herein by reference.

FIELD OF DISCLOSURE

The present disclosure relates generally to blood processing systems including a reusable separation device and a disposable fluid circuit. More particularly, the present disclosure relates to a blood processing system for expelling air from multiple product containers.

BACKGROUND

A wide variety of fluid processing systems are presently in practice and allow for a fluid to be fractionated or separated into its constituent parts. For example, various blood processing systems make it possible to collect particular blood constituents, rather than whole blood, from a blood source. Typically, in such systems, whole blood is drawn from a blood source, the particular blood component or constituent is separated, removed, and collected, and the remaining blood constituents are returned to the blood source in a process commonly referred to as "apheresis." Removing only particular constituents is advantageous when the blood source is a human donor or patient because potentially less time is needed for the donor's body to return to pre-donation levels and donations can be made at more frequent intervals than when whole blood is collected.

When blood is being collected or separated in an apheresis procedure into its various components (such as red blood cells, plasma, platelet-rich plasma, white blood cells), some amount of air invariably is forced into the collection container for the blood or its separated constituents. As is well known, exposure of blood and blood products to air can result in a degradation of the blood/blood product due to, e.g., clotting, coagulation, etc., as well as create an environment conducive to bacterial growth. Additionally, residual air in the blood product may also adversely affect any subsequent processing of the blood product. Consequently, when blood/blood product is being stored in a container for later treatment extracorporeal or for reinfusion to the donor or transfusion to a patient, it is desirable to minimize, and preferably eliminate, any interface within the storage container between the air and the blood/blood product contained therein.

Typically, the residual air will be expelled from the circuit in a process sometimes referred to as "burping." In particular, after collection, product containers associated with a disposable fluid circuit may be burped to ensure residual air is not stored in the product container. If a flexible collection container is used, the air in the collection container is burped by manually squeezing the collection container to force the air back into the disposable fluid circuit.

Certain blood processing procedures include collecting multiple units of a separated blood component. During a multiple unit collection, each unit may be collected in a separate product container. In such instances, each product container would be burped individually. Thus, burping procedures can require several steps such as placing the disposable fluid circuit in varying configurations by clamping particular lines/tubes and tilting and/or inverting containers to suitably burp each product container individually.

Therefore, there exists a need for improved disposable fluid circuits and systems to expel air from multiple product containers.

SUMMARY

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

In one aspect, a system for collecting red blood cells is provided. The system includes a reusable separation device, and the separation device includes a separator. The system also includes a disposable fluid circuit. The disposable fluid circuit includes a leukoreduction filter with a leukoreduction filter inlet and a leukoreduction filter outlet. The disposable fluid circuit also includes a first product container including a first product container inlet and a first product container outlet. The first product container inlet is in fluid communication with the leukoreduction filter outlet. The disposable fluid circuit also includes an openable flow control device located between the leukoreduction filter and the first product container.

Additionally, the disposable fluid circuit includes a second product container including a second product container inlet and a second product container outlet. The second container outlet is in fluid communication with the leukoreduction filter outlet. The disposable fluid circuit also includes a one-way flow control device located between the leukoreduction filter outlet and the second product container, wherein the one-way flow control device is configured to allow fluid flow from the second product container towards the leukoreduction filter. The disposable circuit additionally includes overflow tubing placing the first product container in direct fluid communication with the second product container.

In another aspect, a method of removing air from multiple containers of a collected red blood cell product is provided. The method includes flowing a double unit of separated red blood cell product through a disposable circuit. The disposable circuit includes a leukoreduction filter with a leukoreduction filter inlet and a leukoreduction filter outlet. The disposable circuit also includes a first product container including a first product container inlet and a first product container outlet, and the first container inlet is in fluid communication with the leukoreduction filter outlet. The disposable fluid circuit also includes an openable flow control device configured to be located between the leukoreduction filter and the first product container. The disposable fluid circuit additionally includes a second product container including a second product container inlet and a second product container outlet, and the second product container outlet is in fluid communication with the leukoreduction filter outlet. Additionally, the disposable fluid circuit includes a one-way flow control device located between the leukoreduction filter outlet and the second product container. The one-way flow control device is configured to allow fluid flow from the second product container towards the leukoreduction filter. The disposable fluid circuit also includes overflow tubing placing the first product container in direct fluid communication with the second product container. The method further includes collecting the separated red blood cell product in the first product container, flowing the separated red blood cell product from the first product container to the second product container via the overflow tubing, stopping flow of the red blood cell product to the first product container with the openable flow control device, and flowing the collected red blood cell product in the second product container towards the leukoreduction filter until the red blood cell product reaches the one-way flow control device.

These and other aspects of the present subject are set forth in the following detailed description of the accompanying drawings.

BRIEF DESCRIPTION OF THE DRAWINGS

FIG. 1 is a front perspective view of an example of a blood processing system including a separation device and a disposable fluid circuit mounted thereon.
FIG. 2 is a plan view of an example of a portion of the disposable fluid circuit.
FIG. 3 is a front perspective view of an example of a product container retaining element.
FIG. 4 is a perspective view of a product container removed from the disposable fluid circuit.
FIGS. 5A and 5B are plan views of examples of disposable fluid circuits used in a comparison study described herein.

DETAILED DESCRIPTION OF THE EMBODIMENTS

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

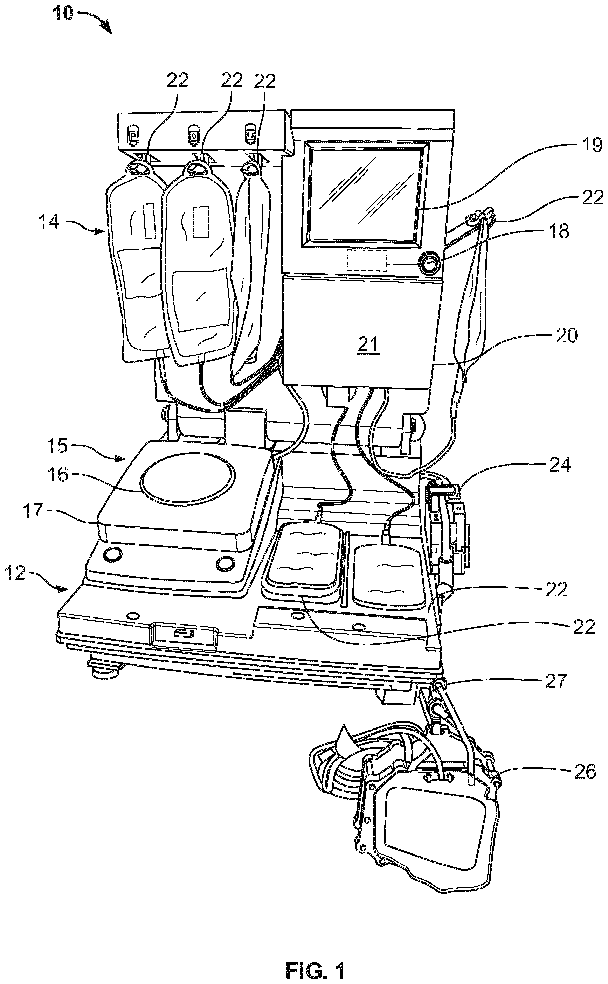

FIG. 1 shows components of an example of a blood or fluid separation system 10. While the system 10 may be referred to herein as a "blood processing system" or a "blood separation system," it will be understood that systems according to the present disclosure can be used for processing a variety of fluids, which may include bodily fluids and non-bodily fluids. In particular, the blood processing system may be configured to separate whole blood into its constituent components including a concentrated red blood cell product. In an embodiment, the blood processing system 10 is a system configured to separate and collect a double unit red blood cell product from whole blood.

Generally, the system 10 may include two principal components, a durable and reusable hardware unit (i.e., blood separation device) 12 and a disposable fluid flow circuit 14. The blood separation device 12 includes a separator 16, additional components that control fluid flow through the disposable fluid circuit 14, and a controller 18, which controls and/or directs the operation of the other components of the blood separation device 12 to perform a blood processing and collection procedure selected by the operator.

Referring to FIG. 1, the figure shows an example of a reusable separation device 12. The blood separation device 12 is configured as a durable item that is capable of repeated use. It will be understood that the blood separation device 12 is merely exemplary of one possible configuration and that blood separation devices according to the present disclosure may be differently configured. Blood separation device 12 can be a blood separation device, such as the ALYX Centrifugal Blood Processing System sold by Fenwal, Inc. of Lake Zurich, Ill., disclosed in more detail in U.S. Patent No. 7,282,154, which is hereby incorporated by reference in its entirety. Other blood separation devices can be used without departing from the scope of the disclosure.

In an embodiment, blood separation device 12 may be a portable device configured to rest on an elevated, generally horizontal support surface, (e.g., a countertop or a tabletop), but it is also within the scope of the present disclosure for the device 12 to include a support base to allow the device to be appropriately positioned and oriented when placed onto a floor or ground surface. While it may be advantageous for the blood separation device 12 to be portable, it is also within the scope of the present disclosure for the blood separation device 12 to be installed in a single location and remain in that location for an extended period of time.

As shown in FIG. 1, blood separation device 12 can include a separation station 15 housing the separator 16. For example, separator 16 can be a centrifuge. Accordingly, separation station 15 may house the centrifuge and/or other components of the separator 16. The separation station 15 may include a lid 17 that is opened to insert and remove a processing chamber of the disposable fluid circuit 14. During a separation procedure, the lid 17 may be closed with a processing chamber of the disposable fluid circuit positioned within the centrifuge as the centrifuge is spun or rotated about an axis. A more detailed description of the separator 16 is disclosed in U.S. Patent No. 7,282,154, which has been previously incorporated herein.

Additionally, as described above, separation device 12 includes a controller 18. In an example, the controller 18 can be housed within the separation device 12. Alternatively, the controller 18 can be an external device associated with the separation device 12. The controller 18 controls the interaction between the components of the device 12 and the components of the disposable fluid circuit 14 to perform a blood processing procedure selected by the operator. Furthermore, in some examples, the separation device 12 can include an interactive user interface 19, which allows the operator to view and comprehend information regarding the operation of the system 10. As shown in FIG. 1, the interface 19 is implemented on an interface screen carried by the separation device 12, which displays information for viewing by the operator in alpha-numeric format and as graphical images. Further details of the controller 18 and the interface 19 can be found in U.S. Patent 7,282,154, previously incorporated herein.

Blood separation device 12 also includes a cassette station 20, which is configured to accommodate a cassette of the disposable fluid circuit 14. In one embodiment, the cassette station 20 is similarly configured to the cassette station of U.S. Patent No. 7,282,154 (which is incorporated herein by reference), but it can be adapted to include additional components and functionality. The cassette station 20 includes a plurality of clamps or valves, which move between a plurality of positions (e.g., between a retracted or lowered position and an actuated or raised position) to selectively contact or otherwise interact with corresponding valve stations of the cassette of the disposable fluid circuit 14. In an example, as shown in FIG. 1, cassette station 20 can include a lid 21 configured to cover the components of the cassette station 20 and the cassette when it is mounted to the separation device 12.

Furthermore, blood separation device 12 can include a variety of sensors configured to monitor the procedures carried out by blood separation device 12. For instance, blood separation device 12 can include weigh stations 22 configured to monitor containers of the disposable fluid circuit 14. As shown in FIG. 1, weigh stations 22 include a series of container hangers/weigh sensors arranged along the top of the separation device 12. Additional "swing-out" hangers/weigh sensors can also be provided on the separation device 12, such as on the side of the separation device 12 as shown in FIG. 1. The weigh stations 22 can also include molded recesses in the base of the separation device 12 to rest containers.

In use, containers are suspended and/or placed on weigh stations 22. As blood or fluids are received into and/or dispensed from the containers during processing, the weigh stations 22 provide output reflecting weight changes over time. This output is conveyed to the controller 18. The controller 18 processes the incremental weight changes to derive fluid processing volumes. The controller 18 generates signals to control processing events based, in part, upon the derived processing volumes.

Additionally, blood separation device 12 can include other sensors such as flow sensors, temperature sensors, a separation interface sensor, and other suitable sensors to monitor a blood processing procedure without departing from the scope of the disclosure.

Blood separation device 12 can also include other retaining elements configured to hold components of the disposable fluid circuit 14. For example, the blood separation device 12 can include a filter holder 24. For instance, depending on the blood separation procedure, the disposable circuit 14 can include a leukoreduction or other type of filter. Filter holder 24 is configured to hold the filter during a blood separation procedure.

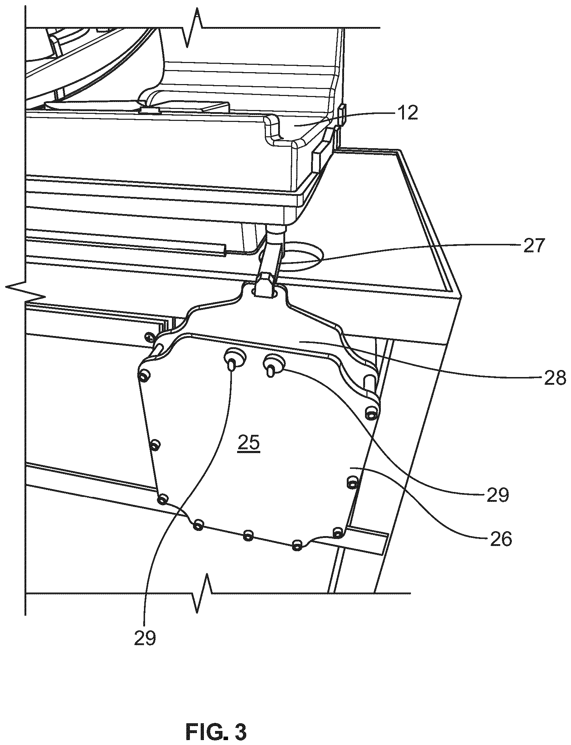

Moreover, the blood separation device 12 includes a product container retaining element 26. In an example, as shown in FIGS. 1 and 3, product container retaining element 26 can be attached to the bottom/base of the separation device 12 with a retaining arm 27. The product container retaining element 26 includes a compartment 28 configured to hold at least one product container. Additionally, product container retaining element 26 can include at least one hook 29 on the outer surface 25 of the product container retaining element 26. The at least one hook 29 is configured for hanging a product container bag. For instance, as shown in FIGS. 1 and 3, product container retaining element 26 includes two hooks 29. The product container retaining element 26 is configured to hold product containers in a configuration, such that the tubing extending from the product containers and ports are oriented at the top of the product container.

The disposable fluid circuit 14 is intended to be a sterile, single use, disposable item. Before beginning a selected blood processing and collection procedure, the operator mounts the various components of the disposable fluid circuit 14 onto device 12 (as FIG. 1 shows). The controller 18 implements the procedure based upon preset protocols, taking into account other input from the operator. Upon completing the procedure, the operator removes the disposable fluid circuit 14 from association with the device 12. The portion of the disposable fluid circuit 14 holding the collected blood component or components are removed from the device and retained for storage, transfusion, or further processing. The remainder of the disposable fluid circuit 14 is removed from the device 12 and can be discarded.

The disposable fluid circuit 14 includes a blood processing chamber, a fluid actuated pump and valve cassette, and an array of associated processing containers and flow tubing coupled to the chamber and cassette. In an example, the disposable fluid circuit 14 includes a blood processing chamber designed for use in association with the separator 16. For instance, the blood processing chamber is loaded into the separation chamber within the separation station 15. In one embodiment, the separator 16 may be a centrifuge. The separation chamber rotates the processing chamber. When rotated the processing chamber centrifugally separates whole blood received from a donor into its component parts, principally, red blood cells, plasma, and intermediate layer called the buffy coat, which is populated by platelets and leukocytes. The configuration of the chamber can vary according to the intended blood separation objectives.

As described above, the disposable fluid circuit also includes a processing cassette, such as a pressure-actuated cassette. In an example, the cassette is the cassette described in U.S. Patent 7,282,154, previously incorporated herein. During a procedure, the cassette is housed within cassette station 20. The cassette provides a centralized, programmable, integrated platform for all pumping and valving functions required for a given blood processing procedure. For example, the cassette includes positive and negative pneumatic pressure, although other types of fluid pressure can be used.

The disposable fluid circuit 14 also includes an array of tubes and containers in flow communication with the cassette and the chamber. The arrangement of tubes and containers can vary according to the processing objectives. For example, the disposable flow circuit can include an array of processing containers such as, but not limited to, an anticoagulant container, a processing fluid (e.g., saline) container, and a plasma collection container, as described in U.S. Patent 7,282,154, which has been previously incorporated by reference herein.

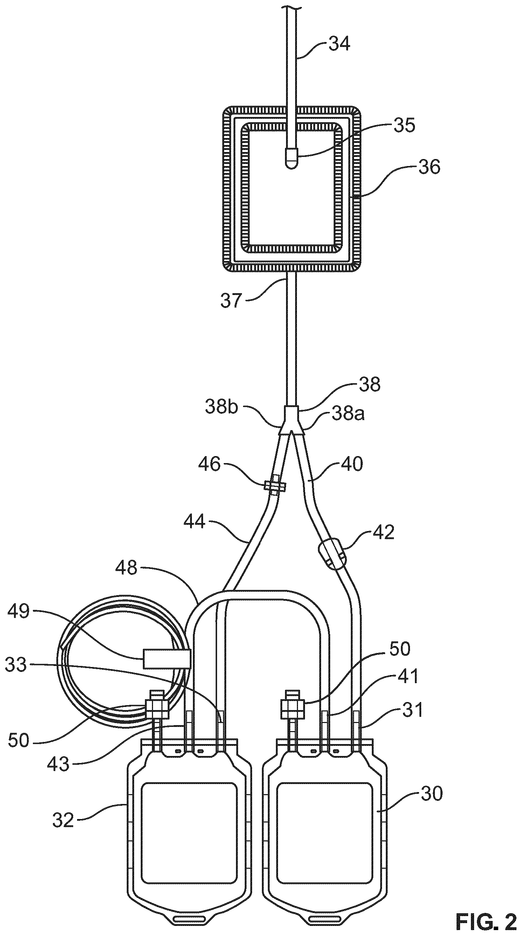

In an example, the system 10 with the disposable fluid circuit 14 is configured to collect a double unit of a separated blood component. Referring to FIG. 2, the figure shows a portion of an example of a disposable fluid circuit 14 with multiple product containers configured to collect a double unit of a separated blood component, such as a double unit of a red blood cell product. Additionally, the disposable circuit shown in FIG. 2 is configured such that air is expelled (i.e., burped) out of the multiple product containers by only requiring burping of a single product container. Although the disposable circuit 14 will be herein described in the context of a double unit red blood cell collection, disposable circuit 14 can be configured to collect various separated blood products and/or fluids without departing from the scope of the disclosure.

As shown in FIG. 2, disposable fluid circuit 14 includes multiple product containers configured to hold a red blood cell product. In particular, disposable circuit 14 includes a first product container 30 and a second product container 32. In an example, first and second product containers 30, 32 are each configured to hold 450 mL of separated red blood cells. The size of the containers may vary depending on the objectives of the procedure, without departing from the scope of the disclosure.

Referring to FIG. 2, the disposable fluid circuit 14 includes a product inlet line 34 in fluid communication with the processing chamber and cassette. After red blood cells have been separated from whole blood, the red blood cells may be flowed through product inlet line 34 towards the product containers 30 and 32. Depending on the blood processing procedure, a leukoreduction filter 36 may be included. For instance, in a red blood cell separation procedure, before entering the product containers 30 and 32, the separated red blood cells may be flowed through a leukoreduction filter 36 located in line with product inlet line 34. In particular, leukoreduction filter 36 includes a leukoreduction filter inlet 35 and a leukoreduction outlet 37 in direct fluid communication with the product inlet line 34.

Leukoreduction filter 36 can be variously constructed without departing from the scope of the disclosure. For instance, leukoreduction filter 36 can include a housing enclosing a filtration medium that can include a membrane or be made from a fibrous material, such as melt blown or spun bonded synthetic fibers (e.g., nylon or polyester or polypropylene), semi-synthetic fibers, regenerated fibers, or inorganic fibers. If fibrous, the medium removes leukocytes by depth filtration. If a membrane, the medium removes leukocytes by exclusion. The housing can include rigid plastic plates sealed about their peripheries. Alternatively, the housing can comprise flexible sheets of medical grade plastic material, such as polyvinyl chloride plasticized with di-2-ethylhexyl-phtalate (PVC-DEHP).

Product inlet line 34 is further in fluid communication with a branch member 38 including a first branch 38a and a second branch 38b. In an example, branch member 38 can be a Y-connector or a T-connector. In another example, branch member 38 can be branched tubing that is integral with the disposable fluid circuit 14.

First product container 30 is in fluid communication with first branch 38a via first product container tubing 40. More particularly, first product container tubing 40 connects the branch 38a to a first product container inlet 31. Additionally, first product container tubing 40 includes an openable flow control device 42. Openable flow control device 42 is configured to stop fluid flow through first product container tubing 40. In an example, openable flow control device 42 can be, but is not limited to, a clamp, roller clamp, or other suitable openable flow control device.

Similarly, second product container 32 is in fluid communication with second branch 38b via a second product container tubing 44. Second product container tubing 44 connects second branch 38b to a second product container outlet 33. Additionally, second product container tubing 44 includes a one-way flow control device 46. One-way flow control device 46 can be, but is not limited to, a check valve. In one embodiment, one-way flow control device 46 is located between second branch 38b and second product container outlet 33. For example, one-way flow control device 46 is located about 1.5 in. from second branch 38b. One-way flow control device 46 is configured to restrict fluid flow from the second branch 38b towards the second product container 32 and allows fluid flow from second product container 32 towards branch member 38.

Disposable fluid circuit 14 further includes overflow tubing 48 connecting first product container 30 to second product container 32 and placing first product container 30 and second product container 32 in direct fluid communication with one another. In particular, overflow tubing 48 connects a first product container outlet 41 to a second product container inlet 43. Excess overflow tubing 48 can be coiled and temporarily attached to itself to ease handling of the disposable fluid circuit 14. For example, excess tubing can be attached to itself with an adhesive tape 49, or other suitable temporary connection. Additionally, overflow tubing 48 can be configured to function as sample collection tubing as will be described herein.

The lengths of the tubing of disposable fluid circuit 14 can vary without departing from the scope of the disclosure. For example, product inlet line 34 between the leukoreduction filter 36 and the branch member 38 can be about 7.125 in., first product container line can be about 10 in., second product container line can be about 9.5 in., and overflow tubing can be about 102.375 in. Additionally, when overflow tubing is sealed and severed to provide sample tubing, each portion of the overflow tubing remaining with either first product container 30 and second product container 32 can be about 51.1875 in.

First product container 30 and second product container 32 can also include various other ports 50. Ports 50 can be used to connect first and second product containers 30, 32 to other tubing, processing circuits, or to access the fluid product within each respective container.

To collect a double unit of red blood cells, disposable fluid circuit 14 is mounted onto the separation device 12. For instance, the processing chamber is loaded into the separation station 15, the cassette is mounted onto cassette station 20, filter 36 is placed into filter holder 24, and various containers are hung or placed on weigh stations 22. Prior to commencing collection, the first and second product containers 30, 32 are placed in association with product container retaining element 26. In particular, the first product container 30 is placed into compartment 28 of retaining element 26 and second product container 32 is hung on hooks 29 of retaining element. When placed in/on product container retaining element 26, the first and second product containers 30, 32 are oriented in an upright configuration such that tubing and ports extend from the top of the containers. Additionally, openable flow control device 42 in the first product container line 40 is placed in an open configuration.

After loading the disposable fluid circuit 14 onto separation device 12, the operator may instruct the pre-programmed controller 18 to carry out a double unit red blood cell collection procedure via interface 19. A more detailed description of loading the disposable fluid circuit 14 and of a double unit red blood cell collection procedure is described in U.S. Patent 7,282,154, which has been incorporated herein.

After whole blood has been separated into its components, a double unit of separated red blood cells can be flowed towards the product containers 30 and 32. In particular, the red blood cells are flowed through product inlet line 34 and through the leukoreduction filter 36 (if present), towards the branch member 38. Because second product container line 44 includes one-way flow control device 46 configured to restrict flow from the second branch 38b towards the second product container 32, the red blood cells flow through branch 38a, through first product container line 40, and into first product container 30.

As the first product container 30 becomes filled, the red blood cells push any residual air out of the first product container 30 and into overflow tubing 48. After the first product container 30 is filled, red blood cells will continue to flow into first product container 30, thus pushing the collected red blood cells (and any residual air) into overflow tubing 48 and into second product container 32.

After second product container 32 is filled with a unit of separated red blood cells, openable flow control device 42 can be placed in a closed configuration to stop fluid flow through first product container line 40. At this point, the second product container 32 can be tilted in a manner to direct residual air in second product container 32 towards the second product container outlet 33 and second product container line 44. Optionally, a second flow control device (not shown), such as, but not limited to, a clamp, can be placed on overflow tubing 48 to restrict fluid flow from the second product container 32 through overflow tubing 48. The second product container 32 can then be (gently) squeezed to expel air out of second product container 32 and into second product container line 44. The second product container 32 is squeezed until the red blood cells reach the one-way flow control device 46, pushing the air through the one-way flow control device 46.

After burping/expelling the air out of the product containers 30 and 32, first product container line 40 and second product container line 44 can be sealed, and the product containers can be removed from the disposable fluid circuit 14. The tubing can be sealed by, for example, welding, heat sealing, or by any other suitable manner. In some instances, the first product container 30 and second product container 32, while still connected by overflow tubing 48, can be hung port-side down (for example, on weigh scales of the separation device 12), allowing for the volume of fluid between the product containers 30, 32 to equalize.

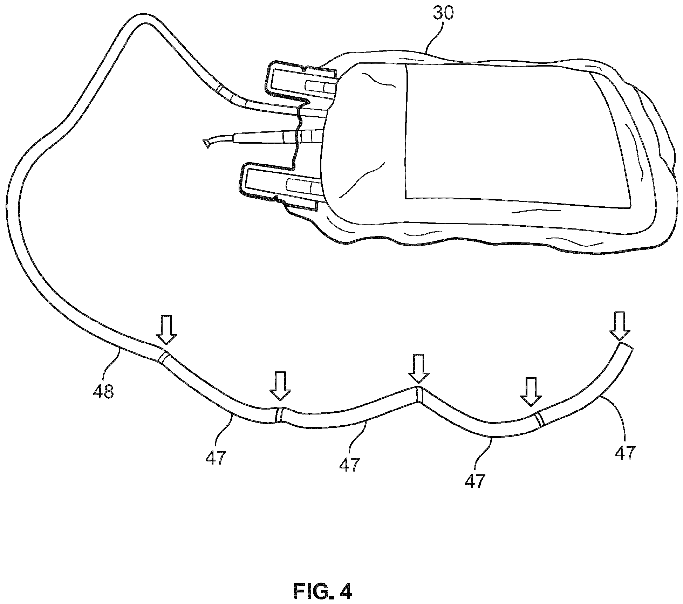

In addition to placing the first product container 30 directly in fluid communication with the second product container 32, overflow tubing 48 can also function as sample collection tubing. After collection, overflow tubing 48 can be sealed and severed at its midpoint, providing the first product container 30 connected to a portion of the overflow tubing 48 and the second product container 32 connected to the remaining portion of the overflow tubing 48. Alternatively, the overflow tubing 48 can be constructed with two printed tube segments joined equidistantly from product containers 30 and 32 with a bushing (not shown). In this instance, the overflow tubing can be sealed and severed at each end of the bushing to provide sample collection tubing connected to the first and second product containers 30, 32. FIG. 4 shows, for illustrative purposes, the first product container 30 and overflow tubing 48 removed from the remainder of disposable fluid circuit 14. The severed overflow tubing 48 can be sealed in intervals and the segments can be removed to provide sample segments 47 including about 0.5 mL of red blood cell product. It is understood that the volume of red blood cell product in each sample segment 47 can vary based on the length of the intervals at which the overflow tubing portions are sealed.

Unrecoverable Fluid Study

Not only is disposable fluid circuit 14 configured to expel air from multiple product containers by burping a single product container, but also, disposable fluid circuit 14 reduces the volume of unrecoverable fluid produced during the burping process. In a study, the volume of unrecoverable fluid in a disposable fluid circuit without a one-way flow control device 46 and overflow tubing 48 was compared to the volume of unrecoverable fluid in a disposable fluid circuit 14 as described herein.

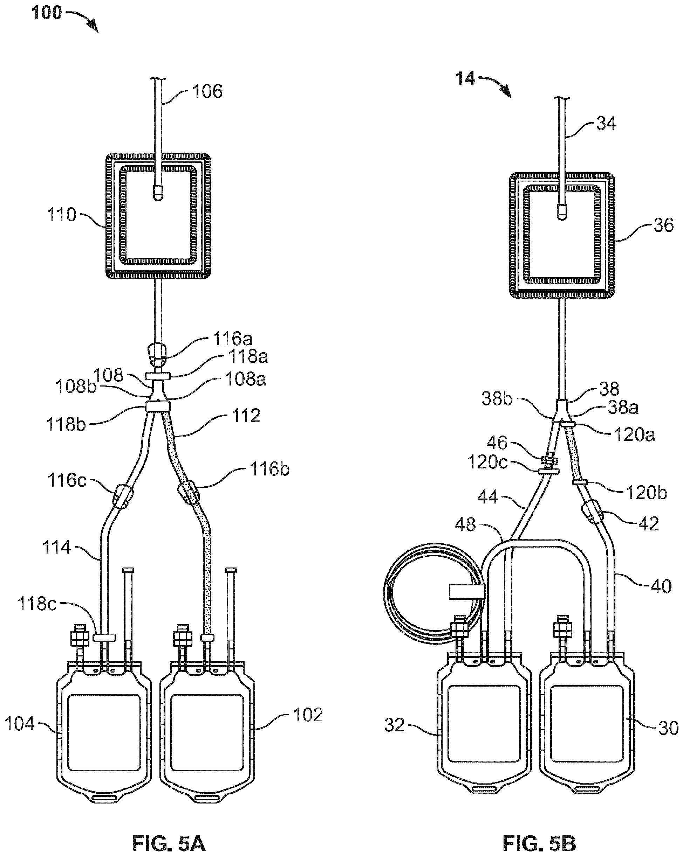

As shown in FIG. 5A, a disposable fluid circuit 100 includes similar elements to disposable fluid circuit 14, except for a one-way flow control device 46 and the overflow tubing 48. For instance, disposable fluid circuit 100 includes a first product container 102 and a second product container 104. Disposable fluid circuit 100 also includes a product inlet line 106 in fluid communication with a branch member 108. A leukoreduction filter 110 is located in line with product inlet line 106. Branch member 108 includes a first branch 108a in fluid communication with a first product container line 112 leading to the first product container 102 and a second branch 108b in fluid communication with a second product container line 114 leading to the second product container 104. Flow control devices 116a, 116b, and 116c, such as clamps, are associated with the product inlet line 106, first product container line 112, and second product container line 114, respectively.

The study was conducted using the ALYX Centrifugal Blood Processing System sold by Fenwal, Inc. of Lake Zurich, Ill., disclosed in more detail in U.S. Patent No. 7,282,154. The separation device was calibrated with calibrated weights and set with the parameters shown in Table 1 below:

Table 1. Study parameters.

| Study Parameters | |
|---|---|
| Sex | Male |
| Height | 85" |
| Weight | 200 lbs. |
| Hematocrit | 45% |
| Whole blood collected | 360 mL |

The disposable fluid circuit 100 was loaded onto the separation device. A plastic beaker was filled with 750 mL of water and a short AV fistula was placed inside to simulate the venipuncture of a donor's arm. The separation device prompts were then followed to initiate a double red blood cell collection procedure. After the procedure, the disposable fluid circuit 100 was burped. To burp the disposable fluid circuit 100, the flow control device 116a was placed in a closed configuration between the branch member 108 and the leukoreduction filter 110. The flow control devices 116b and 116c located on the first product container line 112 and second product container line 114, respectively, were placed in an open configuration. The first product container 102 was then squeezed to expel air towards the second product container 104 through the first product container line 112 and branch member 108. The first product container 102 was squeezed until the first product container line 112 was filled with fluid up to the branch member 108. The first product container line 112 and second product container line 114 were then shut with the flow control devices 116b and 116c, respectively.

Product inlet line 106 was then sealed with a heat seal 118a between the leukoreduction filter 110 and the branch member 108. In particular, the product inlet line 106 was heat sealed between the branch member 108 and the flow control device 116a. After heat sealing product inlet line 106, the first and second product containers 102, 104 were hung on hangers associated with the separation device and the prompts from the device were followed.

The first product container line 112 and the second product container line 114 were then sealed with a heat seal 118b near the branches 108a and 108b, respectively, and the branch member 108 was removed. Additionally, the second product container line 114 was sealed with a heat seal 118c near the second product container 104 and the heat-sealed segment (from the branch member 108 to the second product container 104) was removed.

Disposable fluid circuit 14, as described herein, was prepared similarly. The disposable fluid circuit 14 was loaded onto the separation device. In particular, the first product container 30 and the second product container 32 were placed in association with product container retaining element 26 as described herein. A plastic beaker was filled with 750 mL of water and a short AV fistula was placed inside to simulate the venipuncture of a donor's arm. The separation device prompts were then followed to initiate a double red blood cell collection procedure. After the procedure, the disposable fluid circuit 14 was burped.

To burp the disposable fluid circuit 14, openable flow control device 42 on first product container line 40 was placed in a closed configuration and the filter holder 24 was opened. The second product container 32 was held at an angle such that any trapped air would move towards the one-way flow control device 46. The second product container 32 was then gently squeezed to expel any air into the leukoreduction filter 36. The second product container 32 was squeezed until the fluid reached the one-way flow control device 46.

The first product container line 40 was then sealed with a heat seal 120b between the first branch 38a and the first product container 30, about 3 inches from branch 38a. The first product container line 40 was further sealed with a heat seal 120a at or near first branch 38a. The heat-sealed segment (from branch 38a to about 3 inches from first branch 38a) was then removed from disposable fluid circuit 14. The second product container line 44 was sealed with a heat seal 120c between the one-way flow control device 46 and the second product container 32 at a location near the one-way flow control device 46 (depicted in FIG. 5B just below one-way flow control device 46). The second product container 32 and second product container line 44 were removed from the disposable fluid circuit 14. Additionally, opposite sides of the bushing connected to the overflow tubing 48 were heat sealed and the overflow tubing 48 was removed from the bushing. The first and second product containers 30, 32 were then hung on hangers associated with the separation device and the prompts from the device were followed.

The heat-sealed segments from disposable fluid circuit 100 and disposable fluid circuit 14 were then compared. The lengths and weights of each of the segments were measured and recorded. After obtaining the lengths and weights of the segments, a small portion of each segment was cut on each side of the segments, making sure none of the tubing was lost. An air source was used to expel any fluid out of the segments, and the empty segments were then re-weighed.

Theoretical and actual values of fluid loss were calculated as shown in Tables 2a and 2b below:

Table 2a. Theoretical calculations of fluid loss of disposable fluid circuit 100 and disposable fluid circuit 14 (Figs. 5A and 5B).

| Theoretical Calculations of Fluid Loss | |
| --- | --- |
| Disposable Fluid Circuit 100 (48.25" Tube, Inner Diameter 0.126") | Disposable Fluid Circuit 14 (3.125" Tube, Inner Diameter 0.126") |
| Volume = $\pi r^2 L$ | Volume = $\pi r^2 L$ |
| $Volume = \pi * \left(\frac{0.126\ in}{2}\right) * 48.25\ in$ | $Volume = \pi * \left(\frac{0.126\ in}{2}\right) * 3.125\ in$ |
| Volume = 0.602 in³ | Volume = 0.039 in³ |
| Volume = 9.865 g | Volume = 0.639 g |

Table 2b. Actual calculations of fluid loss of disposable fluid circuit 100 and disposable fluid circuit 14.

| Actual Calculations of Fluid Loss | |
| --- | --- |
| Disposable Fluid Circuit 100 (48.25" Tube, Inner Diameter 0.126") | Disposable Fluid Circuit 14 (3.125" Tube, Inner Diameter 0.126") |
| Total Volume of tube with fluid = 13.17 g | Total Volume of tube with fluid = 1.05 g |
| Volume of empty tube = 12.27 g | Volume of empty tube = 0.85 g |

(continued)

| Actual Calculations of Fluid Loss | |
| --- | --- |
| Total Fluid Loss = 0.90 g | Total Fluid Loss = 0.20 g |

As shown in Tables 2a and 2b above, fluid processing with disposable fluid circuit 14 including one-way flow control device 46 and overflow tubing 48 resulted in less fluid loss as compared to the configuration of disposable fluid circuit 100, in terms of grams of fluid expelled from the tubing segments. Fluid loss of disposable fluid circuit 14, as described herein was 0.20 g, while fluid loss from the disposable fluid circuit 100 was 0.90 g, providing a reduction in fluid loss by 77.8%. By including the one-way flow control device 46 in the second product container line 44 in disposable fluid circuit 14, the disposable fluid circuit 14 increases ease of expelling air and reduces fluid loss by eliminating the need to hold the product container(s) at a certain compression and closing of clamp(s) while holding the product container(s) at a particular compression.

Other Aspects

There are additional aspects to the methods and systems described herein including, without limitation, the following aspects.

Aspect 1. A system for collecting red blood cells including: a reusable separation device, wherein the separation device includes a separator; and a disposable fluid circuit, wherein the disposable fluid circuit includes a leukoreduction filter with a leukoreduction filter inlet and a leukoreduction filter outlet; a first product container including a first product container inlet and a first product container outlet, wherein the first product container inlet is in fluid communication with the leukoreduction filter outlet; an openable flow control device configured to be located between the leukoreduction filter and the first product container; a second product container including a second product container inlet and a second product container outlet, wherein the second container outlet is in fluid communication with the leukoreduction filter outlet; a one-way flow control device located between the leukoreduction filter outlet and the second product container, wherein the one-way flow control device is configured to allow fluid flow from the second product container towards the leukoreduction filter; and overflow tubing placing the first product container in direct fluid communication with the second product container.

Aspect 2. The system of Aspect 1, wherein the reusable separation device includes a product container retaining element configured to hold the first product container and the second product container in an upright configuration.

Aspect 3. The system of Aspect 2, wherein the product container retaining element includes a compartment configured to hold the first product container and hooks on an outer surface of the product container retaining element to hang the second product container.

Aspect 4. The system of any one of Aspects 1-3, wherein the disposable fluid circuit further includes a branch member, wherein the branch member is in fluid communication with the leukoreduction filter, the first product container, and the second product container.

Aspect 5. The system of Aspect 4, wherein the branch member is located between the leukoreduction filter and the openable flow control device and the one-way flow control device.

Aspect 6. The system of any one of Aspects 1-5, wherein the openable flow control device is a clamp.

Aspect 7. The system of any one of Aspects 1-6, wherein the overflow tubing includes sample collection tubing.

Aspect 8. The system of any one of Aspects 1-7, wherein the separation device is a centrifuge.

Aspect 9. A method of removing air from multiple containers of a collected red blood cell product including: flowing a double unit of separated red blood cell product through a disposable circuit, wherein the disposable circuit includes a leukoreduction filter with a leukoreduction filter inlet and a leukoreduction filter outlet; a first product container including a first product container inlet and a first product container outlet, wherein the first product container inlet is in fluid communication with the leukoreduction filter outlet; an openable flow control device configured to be located between the leukoreduction filter and the first product container; a second product container including a second container inlet and a second container outlet, wherein the second container outlet is in fluid communication with the leukoreduction filter outlet; a one-way flow control device located between the leukoreduction filter outlet and the second product container, wherein the one-way flow control device is configured to allow fluid flow from the second product container towards the leukoreduction filter; and overflow tubing placing first product container in direct fluid communication with the second product container; collecting the separated red blood cell product in the first product container; flowing the separated red blood cell product from the first product container to the second product container via the overflow tubing; stopping flow of the red blood cell product to the first product container with the openable flow control device; and flowing the collected red blood cell product in the second product container towards the leukoreduction filter until the red blood cell product reaches the one-way flow control device.

Aspect 10. The method of Aspect 9, further including tilting the second product container to push air towards the one-way flow control device before flowing the collected red blood cell product in the second product container towards the leukoreduction filter.

Aspect 11. The method of Aspect 9, further including closing the overflow tubing with a second flow control device before flowing the collected red blood cell product in the second product container towards the leukoreduction filter.

Aspect 12. The method of any one of Aspects 9-11, further including sealing the first product container and the second product container after flowing the collected red blood cell product in the second product container towards the leukoreduction filter.

Aspect 13. The method of any one of Aspects 9-12, further including placing the first product container and the second product container in a product container retaining element of a reusable separation device to hold the first product container and the second product container in an upright configuration.

Aspect 14. The method of Aspect 13, wherein the retaining element includes a compartment configured to hold the first product container and hooks on an outer surface of the retaining element to hang the second product container.

Aspect 15. The method of any one of Aspects 9-14, wherein the disposable circuit further includes a branch member, wherein the branch member is in fluid communication with the leukoreduction filter, the first product container, and the second product container.

Aspect 16. The method of Aspect 15, wherein the branch member is located between the leukoreduction filter and the openable flow control device and the one-way flow control device.

Aspect 17. The method of any one of Aspects 9-16, wherein the openable flow control device is a clamp.

Aspect 18. The method of any one of Aspects 9-17, wherein the overflow tubing includes sample collection tubing.

Aspect 19. The method of any one of Aspects 13-18, wherein the reusable separation device includes a centrifuge.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

**Claims**

1. A system for collecting red blood cells comprising:

   a reusable separation device, wherein the separation device includes a separator; and
   a disposable fluid circuit, wherein the disposable fluid circuit comprises

   a leukoreduction filter with a leukoreduction filter inlet and a leukoreduction filter outlet;
   a first product container including a first product container inlet and a first product container outlet, wherein the first product container inlet is in fluid communication with the leukoreduction filter outlet;
   an openable flow control device configured to be located between the leukoreduction filter and the first product container;
   a second product container including a second product container inlet and a second product container outlet, wherein the second container outlet is in fluid communication with the leukoreduction filter outlet;
   a one-way flow control device located between the leukoreduction filter outlet and the second product container, wherein the one-way flow control device is configured to allow fluid flow from the second product container towards the leukoreduction filter; and
   overflow tubing placing the first product container in direct fluid communication with the second product container.

2. The system of Claim 1, wherein the reusable separation device includes a product container retaining element configured to hold the first product container and the second product container in an upright configuration.

3. The system of Claim 2, wherein the product container retaining element includes a compartment configured to hold the first product container and hooks on an outer surface of the product container retaining element to hang the second product container.

4. The system of any one of Claims 1-3, wherein the disposable fluid circuit further comprises a branch member, wherein the branch member is in fluid communication with the leukoreduction filter, the first product container, and the second product container.

5. The system of Claim 4, wherein the branch member is located between the leukoreduction filter and the openable flow control device and the one-way flow control device.

6. The system of any one of Claims 1-5, wherein the openable flow control device is a clamp.

7. The system of any one of Claims 1-6, wherein the overflow tubing includes sample collection tubing.

8. A method of removing air from multiple containers of a collected red blood cell product comprising:

flowing a double unit of separated red blood cell product through a disposable circuit, wherein the disposable circuit comprises

a leukoreduction filter with a leukoreduction filter inlet and a leukoreduction filter outlet;
a first product container including a first product container inlet and a first product container outlet, wherein the first product container inlet is in fluid communication with the leukoreduction filter outlet;
an openable flow control device configured to be located between the leukoreduction filter and the first product container;
a second product container including a second container inlet and a second container outlet, wherein the second container outlet is in fluid communication with the leukoreduction filter outlet;
a one-way flow control device located between the leukoreduction filter outlet and the second product container, wherein the one-way flow control device is configured to allow fluid flow from the second product container towards the leukoreduction filter; and
overflow tubing placing first product container in direct fluid communication with the second product container;

collecting the separated red blood cell product in the first product container;
flowing the separated red blood cell product from the first product container to the second product container via the overflow tubing;
stopping flow of the red blood cell product to the first product container with the openable flow control device; and
flowing the collected red blood cell product in the second product container towards the leukoreduction filter until the red blood cell product reaches the one-way flow control device.

9. The method of Claim 8, further comprising tilting the second product container to push air towards the one-way flow control device before flowing the collected red blood cell product in the second product container towards the leukoreduction filter.

10. The method of Claim 8, further comprising closing the overflow tubing with a second flow control device before flowing the collected red blood cell product in the second product container towards the leukoreduction filter.

11. The method of any one of Claims 8-10, further comprising sealing the first product container and the second product container after flowing the collected red blood cell product in the second product container towards the leukoreduction filter.

12. The method of any one of Claims 8-11, further comprising placing the first product container and the second product container in a product container retaining element of a reusable separation device to hold the first product container and the second product container in an upright configuration.

13. The method of Claim 12, wherein the retaining element includes a compartment configured to hold the first product container and hooks on an outer surface of the retaining element to hang the second product container.

14. The method of any one of Claims 8-13, wherein the disposable circuit further comprises a branch member, wherein the branch member is in fluid communication with the leukoreduction filter, the first product container, and the second product container.

15. The method of Claim 14, wherein the branch member is located between the leukoreduction filter and the openable flow control device and the one-way flow control device.

**FIG. 1**

FIG. 2

FIG. 3

**FIG. 4**

FIG. 5A

FIG. 5B

Europäisches
Patentamt

European
Patent Office

Office européen
des brevets

**EUROPEAN SEARCH REPORT**

Application Number

EP 25 15 0030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 2006 026327 A (TERUMO CORP) 2 February 2006 (2006-02-02) * the whole document * * figure 3 * * paragraphs [0029], [0038], [0049], [0055], [0056], [0078] - [0084], [0092] * | 1-15 | INV. A61M1/02 A61M1/36 |
| A | WO 03/033046 A2 (BAXTER INT [US]) 24 April 2003 (2003-04-24) * the whole document * * figures 1, 3 * * page 12, line 6 - line 15 * * page 35, line 3 - page 36, line 19 * | 1-15 | |

TECHNICAL FIELDS
SEARCHED      (IPC)

A61M

The present search report has been drawn up for all claims

| Place of search | Date of completion of the search | Examiner |
|---|---|---|
| The Hague | 8 May 2025 | Pinta, Violaine |

EPO FORM 1503 03.82 (P04C01)

## ANNEX TO THE EUROPEAN SEARCH REPORT
## ON EUROPEAN PATENT APPLICATION NO.

EP 25 15 0030

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2025

| Patent document cited in search report | Publication date | Patent family member(s) | | Publication date |
|---|---|---|---|---|
| JP 2006026327 A | 02-02-2006 | NONE | | |
| WO 03033046 A2 | 24-04-2003 | AU | 2002332008 B2 | 10-07-2008 |
| | | BR | 0213200 A | 23-05-2006 |
| | | CA | 2462718 A1 | 24-04-2003 |
| | | CN | 1568206 A | 19-01-2005 |
| | | EP | 1441800 A2 | 04-08-2004 |
| | | JP | 2005534346 A | 17-11-2005 |
| | | US | 2002131891 A1 | 19-09-2002 |
| | | US | 2005124927 A1 | 09-06-2005 |
| | | WO | 03033046 A2 | 24-04-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63617641

- US 7282154 B